# EUROPEAN PATENT APPLICATION

(11) **EP 2 711 006 A1**
(43) Date of publication of application: **26.03.2014**
(21) Application number: 12185334.5
(22) Date of filing: 21.09.2012
(51) Int. Cl.: A61K 31/465, A61K 31/661, A61K 9/00, A61K 45/06, A61K 9/70, A61P 35/00

(54) **Product Comprising a Nicotine-Containing Material and an Anti-Cancer Agent**

(71) Applicant: Rigas, Basil, Setauket, NY 11733-1504 (US)
(72) Inventor: Rigas, Basil, Setauket, NY 11733-1504 (US)
(74) Representative: Kalhammer, Georg

(57) **Abstract**

The invention is directed to a product comprising a nicotine-containing material and an anti-cancer agent. The product is applicable in the treatment and/or prevention of cancer and precancerous conditions as well as for preventing cancer recurrence.

## Description

### FIELD OF THE INVENTION

The invention is directed to a product comprising a nicotine-containing material and an anti-cancer agent.

### BACKGROUND OF THE INVENTION

Consumption of nicotine-containing products, in particular smoking, is known to affect health and to increase the risk of developing cancer. In particular, the risk of developing lung cancer among smokers is significantly higher than among non-smokers. Lung cancer is the major cause of cancer mortality in the industrial world and its association with smoking is firmly established.

Consumption of tobacco products, in particular smoking, has also been linked to an increased risk of multiple cancers, besides the prototypical case of lung cancer. A detailed analysis of the epidemiological evidence on the association between tobacco smoking and cancer concluded that there is sufficient evidence to establish a causal association between cigarette smoking and cancer of the nasal cavities and paranasal sinuses, nasopharynx, stomach, liver, kidney (renal cell carcinoma) and uterine cervix, and for adenocarcinoma of the oesophagus and myeloid leukaemia (A.J. Sasco et al. Lung Cancer. 2004 Aug 45, Suppl 2, S3-9). These findings add to the previously established list of cancers causally associated with cigarette smoking, namely cancer of the lung, oral cavity, pharynx, larynx, oesophagus, pancreas, urinary bladder and renal pelvis. Other forms of tobacco smoking, such as cigars, pipes and bidis, also increase risk for cancer, including cancer of the lung and parts of the upper aerodigestive tract. Smoking is currently responsible for a third of all cancer deaths in many Western countries. It has been estimated that one in every two smokers will be killed by smoking.

Furthermore, brain cancers such as glioma are also associated with smoking while primary lung cancers are also known to spread to the brain. At least 40% of patients with lung cancer develop brain metastases at some point during their disease. In particular, small cell lung cancer can spread to the brain rapidly, often before the diagnosis of lung cancer is made.

Despite significant advances in its early detection, the survival of lung cancer patients remains poor, with the 5-year survival being as low as 5%. Because of frequent and widespread metastases, surgical procedures for lung cancer are not particularly effective and chemotherapy, the treatment of choice in inoperable cases of lung cancer, has only limited efficacy. The prevention of cancer is currently the only viable option in controlling this dreadful disease.

Smoking cessation would be the most effective method to prevent lung cancer. However, even though it is widely known that smoking causes cancer, smoking is a very difficult addiction to break and currently-marketed smoking cessation products are of limited efficacy. Even patients diagnosed with lung or brain cancer or with precancerous conditions thereof often fail to quit smoking. Thus, there is a pressing need to develop new methods to prevent cancers such as lung and brain cancer in individuals at increased risk of developing these cancers, in particular smokers.

Chemoprevention of cancer, an emerging highly promising approach to cancer prevention, is defined as the administration of an anti-cancer agent, which can comprise a synthetic or a natural compound, to individuals at risk of developing cancer to prevent the development of cancer or to those who already had cancer to prevent its recurrence.

### SUMMARY OF THE INVENTION

The inventor surprisingly found that anti-cancer agents can be advantageously employed for the prevention of cancers, for instance lung and brain cancers and precancerous conditions thereof, when these anti-cancer agents are administered in combination with smoking and/or with another nicotine-containing material described herein. This administration is highly suitable for individuals consuming tobacco products, in particular for smokers. Combining the administration of an anti-cancer agent, particularly of a lung cancer preventing agent with tobacco products including smoking or with smoking cessation products, for instance with nicotine chewing gum, would be very efficient in the prevention of lung cancer or brain cancer or other smoking/tobacco related cancers.

The combination of smoking with the anti-cancer agent maximizes a) the efficacy of the anti-cancer agent, since the anti-cancer agent will be present when the tobacco carcinogens are inhaled, and b) individual compliance in terms of the intake of the anti-cancer agent (which will increase anti-cancer efficacy). Poor compliance with the intake of prescribed medications is well established, especially for long-term administration of disease preventing agents. A case in point is the non-adherence to the use of aspirin among patients post myocardial infarction and other coronary events in whom aspirin was prescribed to prevent another myocardial infarction (E Shantsila, G.Y.H. Lip, Journal of Translational Medicine 2008, 6, 47).

The present invention relates to a product comprising a nicotine-containing material and an anti-cancer agent. The anti-cancer agent may be a compound of natural or synthetic origin. The anti-cancer agent is capable of either preventing or treating cancer or both.

In one embodiment, the anti-cancer agent in the product of the present invention comprises a derivative of a phospho-nonsteroidal anti-inflammatory agent having a phosphate moiety (phospho-NSAIDs). Examples of phospho-NSAIDs include but are not limited to compounds selected from the group consisting of

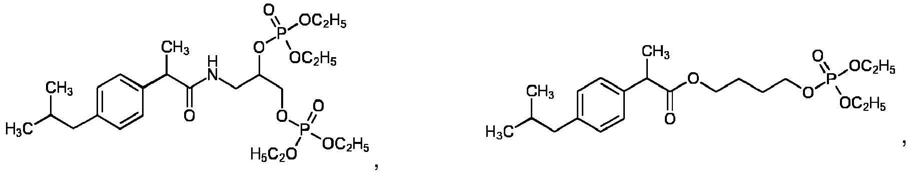

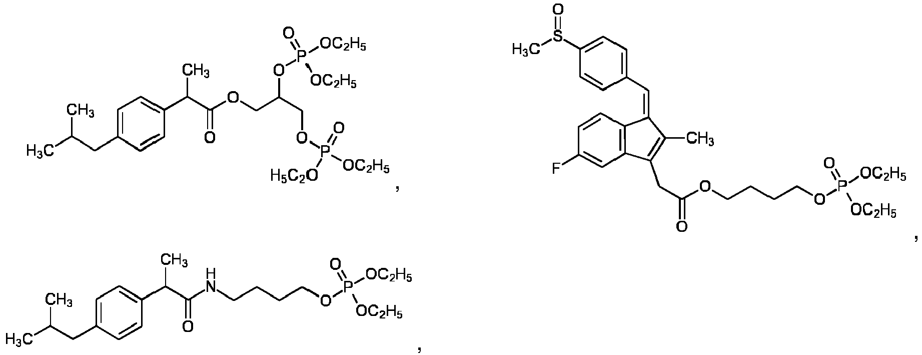

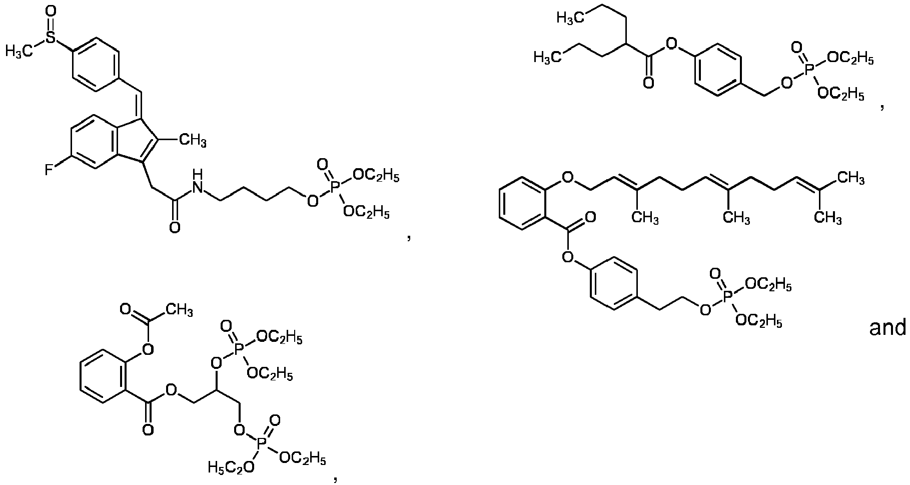

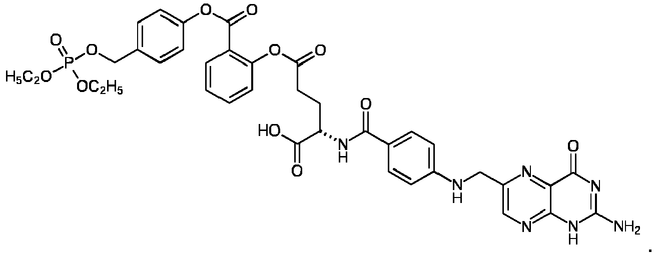

In a further embodiment, the anti-cancer agent in the product of the present invention is an oxidative stress enhancer.

In a further embodiment, the anti-cancer agent may comprise a compound of natural origin such as, for instance, curcumin or other curcuminoids.

In some embodiments of the present invention, the anti-cancer agent comprises one single compound having anti-cancer activity, whereby it is preferred that the anti-cancer agent essentially consists of said compound. In yet other embodiments of the invention, the anti-cancer agent comprises a combination of at least two different compounds having anti-cancer activity. Accordingly, the product of the present invention may comprise a combination of at least two different compounds having anti-cancer activity, for instance a combination of phospho-NSAID and curcumin.

In some preferred embodiments, the nicotine-containing material in the product of the present invention is tobacco leaf.

Preferably, the product of the present invention contains nicotine and the anti-cancer agent in a preferred ratio of from 1000 : 1 to 1 : 10 (wt : wt).

The product of the present invention may be a device capable of delivering both the tobacco product and the anti-cancer agent that can be selected from, but not limited to, the group consisting of cigarette, cigar and smoking pipe, whereby the smoking device may optionally include an additional unit which renders the anti-cancer agent suitable for inhalation. However, the product of the present invention may also be a novel device capable of delivering the tobacco product and the anti-cancer agent.

Alternatively, the product may be a smoking cessation product. Accordingly, the product may be a transdermal patch, an inhalation device, a rectal suppository or an orally applied product.

In still a further embodiment, the product is a smokeless tobacco product.

A further aspect of the invention relates to an anti-cancer agent for use in the prevention and/or treatment of cancer and/or precancerous conditions, wherein said anti-cancer agent is administered simultaneously with nicotine. The cancer may, for instance, be a lung cancer, brain cancer or a precancerous condition thereof.

In a preferred embodiment, the anti-cancer agent is inhaled together with tobacco smoke.

### BRIEF DESCRIPTION OF THE FIGURES

**Figures 1A-1F****.** Smoking devices comprising a nicotine-containing material and an anti-cancer agent.
**Figure 2****.** Levels of phospho-sulindac (PS **V**) and its metabolites in the lungs (A) and plasma (B) of mice subjected to aerosol administration of PS **V.**
**Figure 3****.** Survival rates of control and aerosolized-PS treated groups of mice implanted orthotopically with A549 cells.
**Figure 4****.** Aerosol administration of PS **V** prevents lung tumorigenesis.
**Figure 5****.** Aerosol administration of PS **V** prevents lung tumorigenesis.
**Figure 6****.** Lung level of PS **V** after inhalation and oral administration.
**Figure 7****.** Plasma level of PS **V** after inhalation and oral administration.

### DETAILED DESCRIPTION

The present invention provides a novel product comprising a nicotine-containing material and an anti-cancer agent.

The term "anti-cancer agent" refers to a natural or synthetic agent that is capable of either preventing or treating cancer or both. Preferably, the anti-cancer agent is capable of inhibiting the proliferation or preventing the development of cancer cells.

In some embodiments of the present invention, the anti-cancer agent comprises a combination of at least two different compounds having anti-cancer activity. Accordingly, the product of the present invention may comprise a combination of at least two different compounds having anti-cancer activity. For instance, the product may contain two different compounds having anti-cancer activity in the ratio of from 10 : 1 to 1 : 10, more preferred from 7 : 1 to 1 : 7, particularly preferred from 4 : 1 to 1 : 4, for instance 1 : 1 (weight : weight). As an example, a combination of curcumin and a phospho-NSAID can be mentioned.

Preferred anti-cancer agents are capable of inhibiting the growth or preventing the development of solid tumors *in vivo.* Preferred anti-cancer agents are also capable of reducing the size of a solid tumor *in vivo.*

The anti-cancer agent may comprise compounds including, but not being limited to, androgen inhibitors, such as flutamide and luprolide; antiestrogens, such as tamoxifen; antimetabolites and cytotoxic agents, such as daunorubicin, fluorouracil, floxuridine, interferon alpha, methotrexate, plicamycin, mecaptopurine, thioguanine, adriamycin, carmustine, lomustine, cytarabine, cyclophosphamide, doxorubicin, estramustine, altretamine, hydroxyurea, ifosfamide, procarbazine, mutamycin, busulfan, mitoxantrone, streptozocin, bleomycin, dactinomycin, and idamycin; hormones, such as medroxyprogesterone, estramustine, ethinyl estradiol, estradiol, leuprolide, megestrol, octreotide, diethylstilbestrol, chlorotrianisene, etoposide, podophyllotoxin, and goserelin; nitrogen mustard derivatives, such as melphalan, chlorambucil, methlorethamine, and thiotepa, steroids, such as betamethasone; differentiation-inducing agents, such as retinoic acid, vitamin D, cytokines; and other antineoplastic agents, such as platinum compounds, dicarbazine, asparaginase, leucovorin, mitotane, vincristine, vinblastine, and taxanes (e.g., taxol, paclitaxel, docetaxel). A list of anti-cancer agents can be found in L. Brunton, B. Chabner and B. Knollman (eds). Goodman and Gilman's, The Pharmacological Basis of Therapeutics, Twelfth Edition, 2011, McGraw Hill Companies, New York, NY.

In one preferred embodiment, the anti-cancer agent comprises a tyrosine kinase inhibitor (TKI). A TKI inhibits the tyrosine kinase activity of at least one tyrosine kinase. The inhibition may be reversible or irreversible. TKIs include, but are not limited to, compounds such as imatinib, dasatinib, nilotinib, gefitinib, erlotinib, lapatinib, sunitinib, sorafenib and pazopanib. Various TKIs are, for instance, described in Hartmann *et al.* (J. Th. Hartman et al. Cur. Drug Metab, 2009, 10, pp. 470-481).

In another embodiment, the anti-cancer agent comprises a compound having oxidative stress-inducing ability. This compound increases the oxidative stress of cancer cells by inhibiting the mechanisms that cancer cells utilize to compensate for reactive oxygen species (ROS) and/or activating cellular signaling pathways that lead to cytotoxicity. Examples of the corresponding compounds include platinum formulation such as cis-platin, carboplatin, and oxaliplatin, cyclophosphamide, fluorouracil, etoposide, thiostrepton, doxorubicin, bleomycin, and mitomycin. The term "reactive oxygen species" relates to highly reactive metabolites of molecular oxygen, which are generated in a tissue environment. ROS can be free radicals, ions or molecules. Examples of ROS include, but are not limited to, superoxide ion radical (O₂⁻ ), hydroxyl radical (HO^{.}), peroxide (ROO^{.}), alkoxyl radicals (RO^{.}), hydrogen peroxide (H₂O₂), organic peroxide (ROOR'), ozone (O₃), singlet oxygen (¹O₂), etc.

Further preferred anti-cancer agents for use in the present invention may comprise a compound such as difluoromethylornithine or erlotinide.

In a further embodiment, the anti-cancer agent comprises at least one derivative of a phospho-nonsteroidal anti-inflammatory agent having a phosphate moiety (phospho-NSAIDs). The corresponding compounds for use in the present invention are disclosed in WO 2009/023631, WO 2005/065361, and WO 2011/094589. These compounds are incorporated herein by reference. Further incorporated herein are the compounds disclosed in the US provisional application titled "COMPOUNDS AND COMPOSITIONS FOR USE IN THE TREATMENT AND PREVENTION OF LUNG AND BRAIN CANCER AND PRECANCEROUS CONDITIONS THEREOF", filed concurrently with the present application. Particularly preferred for this purpose are phospho-ibuprofen I, phospho-ibuprofen glycerol **II**, phospho-ibuprofen glycerol amide **III**, phospho-ibuprofen amide **IV**, phospho-sulindac V, phospho-sulindac amide **VI,** phospho-aspirin **VII,** phospho-valproic acid **VIII,** and the compounds **IX** and **X**, the structures of which are shown below:

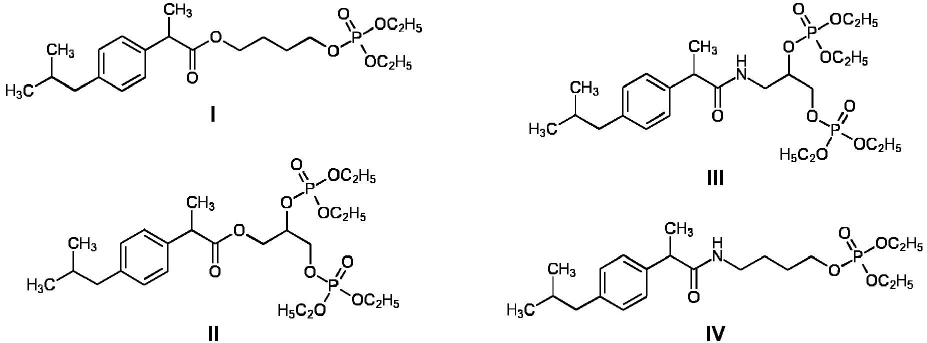

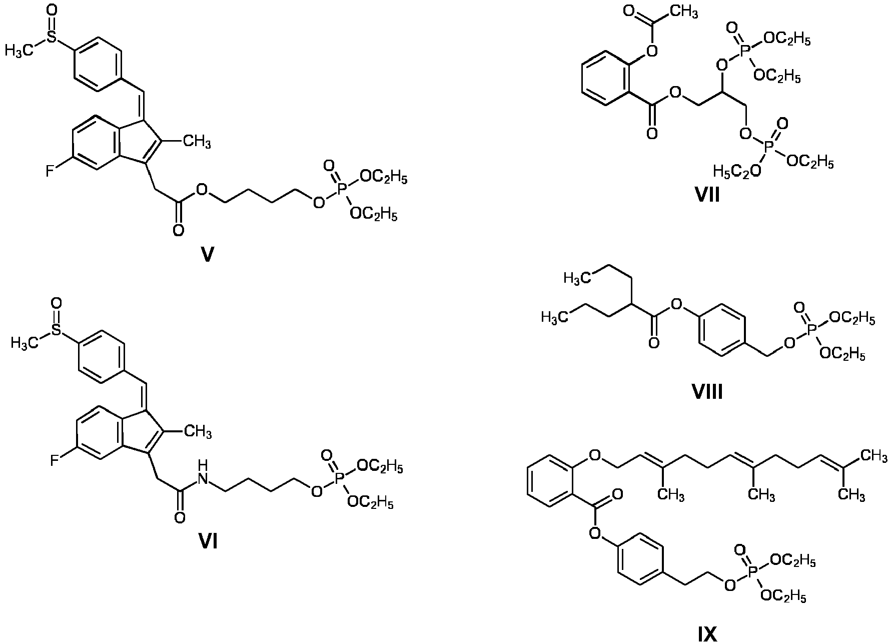

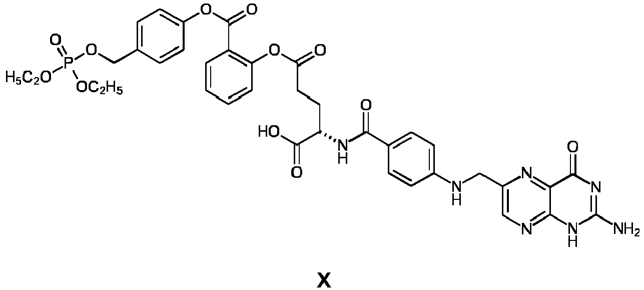

The choice of the nicotine-containing material for use in the product of the present invention is not particularly limited. Preferably, the nicotine-containing material used is the leaf of a tobacco plant i.e. a plant of the genus *Nicotiana,* such as *Nicotiana tabaccum.* Tobacco leaves of several types may be employed. Suitable types of tobacco leaves include, but are not limited to, Brightleaf tobacco, Burley, Cavendish, Corojo, Criollo, Oriental tobacco, Perique, Shade tobacco, Thuoc lao, Type 22, White Burley, wild tobacco and Y1.

The content of the nicotine-containing material and of the anti-cancer agent in the product can be readily chosen by a person skilled in the art to provide an advantageous therapeutic effect. Preferably, the product contains nicotine and the anti-cancer agent in the ratio of from 1000 : 1 to 1 : 10, more preferred from 10 : 1 to 1 : 10, even more preferred from 7 : 1 to 1 : 7, particularly preferred from 4 : 1 to 1 : 4, for instance 1 : 1 (weight: weight).

Alternatively, the nicotine-containing material may be nicotine (IUPAC name: (S)-(-)-3-(1-methylpyrrolidin-2-yl)pyridine) or a pharmaceutically acceptable salt thereof. S.M. Berge et al. describe pharmaceutically acceptable salts in detail in J. Pharmaceutical Sciences, 1977, 66, 1-19, incorporated herein by reference. Examples of pharmaceutically acceptable acid addition salts can be formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts and coformer molecules for cocrystal formation include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like. Nicotine may be further bound to a polymeric material such as an ion-exchange resin, for instance to polymethacrilic acid, such as Amberlite^{®} IRP64. The corresponding material is commercially available under the name Nicotine Polacrilex.

Preferably, one dosage of the product contains nicotine from 0.01 to 100 mg, preferably from 0.1 to 10 mg, more preferred from 0.5 to 7 mg, yet even more preferred from 0.7 to 5 mg and particularly preferred from 1 to 3 mg nicotine. Depending on the intended mode of administration, the product of the present invention may additionally comprise a pharmaceutically acceptable carrier which, as used herein, includes solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E.W. Martin (Mack Publishing Co., Easton, Pa., 1980) discloses various carriers used in formulating pharmaceutical compositions and known techniques for the preparation thereof. Some examples of materials which can serve as pharmaceutically acceptable carriers include, but are not limited to, volatile solid materials, such as menthol, sugars such as lactose, glucose and sucrose; excipients such as cocoa butter; oils such as peanut oil, cottonseed oil; safflower oil, sesame oil; olive oil; corn oil and soybean oil; glycols; such as propylene glycol; esters such as ethyl oleate and ethyl laurate; agar; natural and synthetic phospholipids, such as soybean and egg yolk phosphatides, lecithin, hydrogenated soy lecithin, dimyristoyl lecithin, dipalmitoyl lecithin, distearoyl lecithin, dioleoyl lecithin, hydroxylated lecithin, lysophosphatidylcholine, cardiolipin, sphingomyelin, phosphatidylcholine, phosphatidyl ethanolamine, diastearoyl phosphatidylethanolamine (DSPE) and its pegylated esters, such as DSPE-PEG750 and DSPE-PEG2000, phosphatidic acid, phosphatidyl glycerol and phosphatidyl serine. Commercial grades of lecithin which are preferred include those which are available under the trade name Phosal^{®} or Phospholipon^{®} and include Phosal^{®} 53 MCT, Phosal^{®} 50 PG, Phosal^{®} 75 SA, Phospholipon^{®} 90H, Phospholipon^{®} 90G and Phospholipon^{®} 90 NG; soy-phosphatidylcholine (SoyPC) and DSPE-PEG2000 are particularly preferred; buffering agents such as amino acids; pyrogen-free water; isotonic saline; Ringer's solution; ethyl alcohol, and phosphate buffer solutions, as well as other non-toxic compatible lubricants such as sodium lauryl sulfate as well as releasing agents, coating agents, sweetening, flavoring and perfuming agents, preservatives and antioxidants can also be present in the product of the present invention.

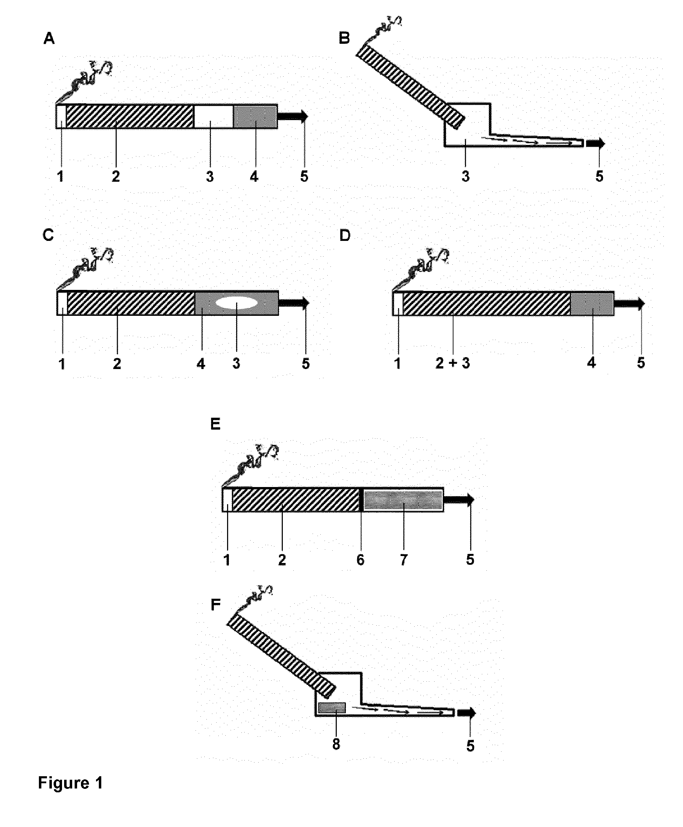

In some embodiments, the product for the present invention is a smoking device such as cigarette, cigar or smoking pipe. In these embodiments, the anti-cancer agent is inhaled at the same time that the smoker smokes. For this purpose, the nicotine-containing material and the anti-cancer agent can be, for instance, incorporated in a cigarette, a cigar (see Figure 1A) or in a smoking device such as a smoking pipe (for instance, in the chamber of a smoking pipe, see Figure 1 B) or in a water pipe, etc. In addition, the smoking device may optionally comprise an additional unit which renders the anti-cancer agent suitable for inhalation so that smoke and the anti-cancer agent can be inhaled simultaneously or in sequence. The additional unit may, for instance, be a pressurized aerosol spray dispenser, a nebulizer, an atomizer or a cartonizer.

The term "smoking" as used herein refers to the action of inhaling or tasting the smoke of burning plant material, preferably of tobacco leaves. Smoking further includes a process wherein the smoking composition is heated but not pyrolysed, and the heated vapors are inhaled or tasted by the smoker.

Figure 1A: the anti-cancer agent or a pharmaceutical composition thereof **3** is incorporated into the cigarette containing tobacco **2** and, optionally, having a filter **4.** Tobacco smoke coming from the pyrolysis zone **1** causes volatilization of the anti-cancer agent. In order to improve volatilization the anti-cancer agent can be formulated with a volatile solid such as menthol. The tobacco smoke 5 containing the anticancer agent enters the mouth and the lungs of the smoker.

Figure1B: the anticancer agent or a pharmaceutical composition thereof **3** is incorporated into a smoking pipe. Alternatively, another smoking device such as a water pipe can be employed. The volatilization of the anti-cancer agent can be additionally facilitated by external heating, for instance, by using an electric heating element.

Figure 1C: a further embodiment of the present invention is shown. The anti-cancer agent is administered in a so-called "cigarette with menthol capsule". The anti-cancer agent or a pharmaceutical composition thereof **3** is incorporated in a menthol capsule which, in turn, is located in the filter **4.** Cigarettes with menthol capsules are known in the prior art and are, for instance, described in US 2009/0277465. The anticancer agent or a pharmaceutical composition thereof is incorporated into the menthol capsule and is volatilized during the smoking process. Thus, this embodiment is particularly suited for smokers and aims to prevent lung cancer and/or precancerous conditions in the lung.

Figure 1 D: a further embodiment is shown. The anti-cancer agent or a pharmaceutical composition thereof **3** is directly mixed with tobacco **2.** Thus, volatilization the anti-cancer agent occurs primarily in the pyrolysis zone **1** of the cigarette and the tobacco smoke **5** containing the anti-cancer agent enters the mouth and the lungs of the smoker. In this embodiment, the filter **4** is optional. This embodiment is particularly useful, if the anti-cancer agent is sufficiently volatile.

Figure 1 E: a further embodiment is shown. The anti-cancer agent or a pharmaceutical composition thereof **3** (not shown) is incorporated in an electronic cigarette cartridge **7.** The cartridge **7** may be designed as an atomizer or as a cartonizer. Valve **6** prevents the entry of the aerosol and solvent vapor emitted by the cartridge **7** into the tobacco section **2.** In this embodiment, tobacco smoke formed in the pyrolysis zone **1** enters the section containing the electronic cigarette cartridge **7** via the valve **6.** Thus, the aerosol emitted by the electronic cigarette cartridge **7** is mixed with the tobacco smoke and the resulting mixture **5** is subsequently inhaled by the smoker.

Figure 1 F: a further embodiment is shown. The anti-cancer agent or a pharmaceutical composition thereof 3 (not shown) is incorporated in an additional unit **8** which may be an atomizer or cartonizer or similar device that renders the anti-cancer agent suitable for inhalation. Having an appropriate valve or other mechanism(s), smoke and inhalable agent may be mixed to simultaneously deliver smoke and anti-cancer agent to the mouth and ultimately the lungs of the smoker.

In all embodiments shown in Figures 1A-1F the smoker also inhales the anti-cancer agent during inhalation of the tobacco smoke. In order to facilitate volatilization of the anti-cancer agent, it can be formulated in a dry powder aerosol composition such as the one described by Plumley C, et al. (Int. J. Pharm. 369, (1-2), pages 136-143, 2009) or in a pharmaceutical composition containing volatile solids such as menthol. Alternatively, the neat anti-cancer agent can be used instead of the pharmaceutical composition thereof.

In other embodiments, the product of the present invention is a smoking cessation product such as, for instance, transdermal patch, inhalation device, orally applied product or rectal suppository.

In one embodiment of the present invention, the product is a transdermal patch. Transdermal patches comprising a nicotine-containing material are known in the prior art and are, for instance, described in US 2009/0246264, which is incorporated here by reference. Preferably, the transdermal patch simultaneously delivers nicotine and the anti-cancer agent to the patient. According to the present invention, said transdermal patch preferably releases more than 30 wt.-%, more preferably more than 50 wt.-% and particularly preferred more than 70 wt.-% of its total content of the anti-cancer agent within 24 h to the skin of the patient. The nicotine-containing material and the anti-cancer agent may be present in separate layers of the transdermal patch or as a mixture in the same layer. The layers containing the nicotine-containing material, the anti-cancer agent or a mixture thereof typically contain gelling agents such as homo- or copolymers of 2-hydroxyethyl acrylate or 2-hydroxyethyl methacrylate, poly(vinyl alcohol), Pluronic^{®}, carboxymethyl cellulose, hydroxyethyl starch, hydroxypropyl cellulose or methyl cellulose. These layers may further contain suitable penetration enhancers such as dimethyl sulfoxide, N,N-dimethylacetamide, triglycerides (e.g. soybean oil), unsaturated oils, aloe compositions (e.g. aloe vera gel), octalylphenylpolyethylene glycol, oleic acid, polyethylene glycol 400, propylene glycol, n-decyl methyl sulfoxide, fatty acid esters (e.g. isopropyl myristate, methyl laurate, glycerol monooleate and propylene glycol monooleate) and N-methyl pyrrolidone or mixtures thereof.

In another embodiment of the present invention, the product is an inhalation device. The inhalation device may be a smoking device, a mechanical device for pulmonary delivery, a device for the nasal anti-cancer agent delivery or a so-called electronic cigarette. Mechanical devices for pulmonary delivery of the nicotine-containing material and the anti-cancer agent include, but are not limited to, nebulizers, metered dose inhalers, and powder inhalers, all of which are familiar to those skilled in the art. Some specific examples of commercially available devices suitable for the practice of this invention are the Ultravent nebulizer (Mallinckrodt, Inc., St. Louis, Mo, USA), the Acorn II^{®} nebulizer (Marquest Medical Products, Englewood, Colorado, USA), the Ventolin^{®} metered dose inhaler (Glaxo Inc., Research Triangle Park, N.C. USA) and the Spinhaler powder inhaler (Fisons Corp, Bedford, Mass. USA).

Devices for nasal anti-cancer agent delivery are also known to persons skilled in the art and are commercially available, for instance, from Bespak (Bespak Europe Limited, United Kingdom).

In some other embodiments, the pharmaceutical composition of the present invention is directly heated, whereby nicotine and the anti-cancer agent form a vapor and subsequently condense into an aerosol. Thus, an aerosol containing nicotine and the anti-cancer agent is formed. Subsequently, the patient inhales this aerosol. Suitable devices are known in the prior art and are, for instance, described in US 2003/0000518.

Alternatively, the combination of the nicotine-containing material and the anti-cancer agent may be administered in a so-called electronic cigarette. Such devices are known in the prior art and are, for instance, described in US 2006/0196518, US 2007/0267031 and Caponnetto et al. (Journal of Medical Case Reports 5, 585, 2011). An electronic cigarette is primarily used for the administration of nicotine and, optionally, of flavors such as menthol. Incorporating of the nicotine-containing material and the anti-cancer agent in the cartridge allows their efficient administration by the respiratory route. Advantageously, said cartridge can be employed in a commercially available electronic cigarette. The cartridge may be an atomizer or a cartonizer, as known in the prior art.

Accordingly, in a further embodiment of the present invention, the product is a cartridge comprising the nicotine-containing material and the anti-cancer agent for use in an electronic cigarette. Such cartridge can also be used by patients suffering from lung cancer or those with precancerous conditions of the lung.

The smoking cessation product may be in the form of a pressurized aerosol spray dispenser, which contains a suitable propellant, e.g. hydrofluoroalkanes, chlorofluorocarbons, carbon dioxide, or a nebulizer. In this embodiment, the dosage unit may be determined by providing a valve to deliver a metered amount. Capsules and cartridges of e.g. gelatine for use in an inhaler or insufflator may be formulated containing a powder mix of the nicotine-containing material, the anti-cancer agent and a suitable pharmaceutically acceptable carrier.

Administration by the respiratory route usually requires the use of pharmaceutical compositions suitable for the dispensing of the nicotine-containing material and the anti-cancer agent. Typically, each pharmaceutical composition is specific to the type of device employed and may involve the use of an appropriate propellant material, in addition to the usual diluents, adjuvants and/or carriers. Also, the use of liposomes, microcapsules or microspheres, inclusion complexes, micelles or other anti-cancer agent nanocarriers, or other types of carriers is contemplated. The combination of the nicotine-containing material and the anti-cancer agent may be prepared in different pharmaceutical compositions depending on their physical and chemical properties or the type of device employed.

Pharmaceutical composition suitable for use with a nebulizer, either jet or ultrasonic, will typically comprise the nicotine-containing material, preferably nicotine or a pharmaceutically acceptable salt thereof, and the anti-cancer agent dissolved in a solvent and containing typically about 0.1 to 25 mg of the anti-cancer agent per 1 ml of solution. The pharmaceutical composition may also include a buffer, for instance, an amino acid, and a simple sugar (e.g. for stabilization of the anti-cancer agent and regulation of osmotic pressure). The solvent in the pharmaceutical composition may be selected from the group consisting of water, ethanol, 1,3-propylene glycol, glycerol or a mixture of any of those. Nebulized pharmaceutical compositions may also contain a surfactant, to reduce or prevent surface induced aggregation of the nicotine-containing material or of the anti-cancer agent caused by atomization of the solution in forming the aerosol.

Pharmaceutical compositions for use with a metered-dose inhaler device generally comprise a finely divided powder containing the nicotine-containing material and the anti-cancer agent (or a pharmaceutically acceptable derivative thereof) suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Pharmaceutical compositions for dispensing from a powder inhaler device will comprise a finely divided dry powder containing the nicotine-containing material and the anti-cancer agent and may also include a bulking agent, such as lactose, sorbitol, sucrose, or mannitol in amounts which facilitate dispersal of the powder from the device, e.g. 50 to 90% by weight of the formulation. The nicotine-containing material and the anti-cancer agent should most advantageously be prepared in a particulate form with an average particle size of less than 10 µm, preferably less than 5 µm and more preferred less than 1 µm, for effective delivery to the distal lung.

In a further embodiment of the present invention, the product is a rectal suppository. In this embodiment, the nicotine-containing material and the anti-cancer agent are mixed with suitable non-irritating excipients or carriers such as cacao butter, polyethylene glycol or a suppository wax, which are solid at ambient temperature but liquid at body temperature and therefore melt in the rectum cavity and release nicotine and the anti-cancer agent.

In yet a further embodiment of the present invention, the product is an orally applied product. Thus, for instance, the product may be in the form of a chewing gum. Nicotine chewing gums are known in the prior art, are described in US 2010/0130562 and are commercially available under the trade names such as Nicorette^{®} and Thrive^{®}. In this embodiment, the product of the present invention contains the nicotine-containing material, the anti-cancer agent as well as the chewing gum base, plasticizers, buffering agents, sweeteners, antioxidants, flavoring agents and colorants. Examples of suitable plasticizers include lecithin, lanoline, glycerides, stearic acid, sodium stearate, potassium stearate or waxes such as bee wax. Examples of sweeteners that may be used in the product of the present invention include saccharides as well as salts of saccharine or cyclamic acid as well as sugar alcohols such as sorbitol, mannitol, or xylitol. The flavoring agents for use in the product of the present invention may include, without limitation, the flavors of cherry, cinnamon, grape, apple, lemon, orange, peppermint, raspberry, strawberry, chocolate, and the like.

In a further embodiment, the orally applied product is in the form of smokeless tobacco. In this embodiment, the smokeless tobacco is formulated with the anti-cancer agent and further contains plasticizers as well as sweeteners and flavoring agents described above. Smokeless tobacco products include, but are not limited to, dipping tobacco, chewing tobacco, snuff, snus, creamy snuff, tobacco gum, gutkha, gul, khaini, qiwam, mawa, mishri, pan masala and zarda, chewing tobacco being particularly preferred.

Another aspect of the present invention relates to the products described herein for use in the treatment and/or prevention of cancer and/or precancerous conditions. "Cancer" as used herein refers to an uncontrolled growth of cells which interferes with the normal functioning of the bodily organs and systems. Cancers include, but are not limited to, basal cell carcinoma, biliary tract cancer, bladder cancer, bone cancer, brain and other central nervous system (CNS) cancer, breast cancer, cervical cancer, choriocarcinoma, colon and rectum cancer, connective tissue cancer, cancer of the digestive system, endometrial cancer, esophageal cancer, eye cancer, cancer of the head and neck, gastric cancer, intra-epithelial neoplasm, kidney cancer, larynx cancer, leukemias, including hairy cell leukemia, liver cancer, lung cancer (e.g. small cell and non-small cell), lymphomas including Hodgkin's and non-Hodgkin's lymphomas, melanoma, myeloma, neuroblastoma, oral cavity cancer (e.g. lip, tongue, mouth, and pharynx), ovarian cancer, pancreatic cancer, prostate cancer, retinoblastoma, rhabdomyosarcoma, rectal cancer, renal cancer, cancer of the respiratory system, sarcoma, skin cancer, stomach cancer, testicular cancer, thyroid cancer, uterine cancer, cancer of the urinary system, as well as other carcinomas and sarcomas.

In yet another embodiment, the product of the present invention is useful in the treatment and/or prevention of cancer and precancerous conditions, including, but not limited to, benign prostatic hypertrophy, colon adenomas, actinic keratosis and various premalignant conditions of the lung, breast and pancreas.

The anti-cancer agent and pharmaceutical compositions thereof inhibit the growth of human or animal cancer cell lines such as A549 human lung cancer cells in *in vitro* tests and have IC₅₀ value of preferably less than 800 µM, more preferred of less than 400 µM, particularly preferred of less than 70 µM. The tests are preferably carried out as specified in S. Joseph et al. (Molecular Medicine Reports 2011, 4, 891-899).

One embodiment of the present invention relates to a method for preventing cancer by means of administering the product of the present invention. Accordingly, treatment of an individual with the product of the present invention reduces the risk of the individual to develop cancer. Preferably, after the treatment, the risk of the individual to develop cancer is reduced by 5% or greater; more preferably, the risk develop cancer is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. As used herein, reducing risk of developing cancer includes decreasing the probability or incidence of developing cancer for an individual compared to a relevant, e.g. untreated, control population, or in the same individual prior to treatment according to the invention. Reduced risk of developing cancer may include delaying or preventing the onset of a cancer. Risk of developing cancer can also be reduced if the severity of a cancer or a precancerous condition is reduced to such a level that it is not of clinical relevance. That is, the cancer or a precancerous condition may be present but at a level that does not endanger the life, activities, and/or well-being of the individual. For example, a small tumor may regress and disappear, or remain static. Preferably, tumor formation does not occur. In some circumstances the occurrence of the cancer or the precancerous condition is reduced to the extent that the individual does not present any signs of the cancer or the precancerous condition during and/or after the treatment period.

The method for preventing cancer according to the present invention is beneficial both for individuals having a precancerous condition and individuals who are healthy. Individuals with lifestyle habits that could lead to cancer, particularly smokers, and individuals affected by diseases for which the probability of cancer incidence is high have a particularly high order of priority as individuals for the preventive method of the present invention. Furthermore, individuals who are likely to acquire familial cancers, and such individuals as those who are diagnosed with a risk of cancer by means of gene diagnoses based on single-nucleotide polymorphism or the like may also be targeted.

Treating cancer can result in a reduction in size of a tumor. A reduction in size of a tumor may also be referred to as "tumor regression." Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. The size of a tumor may be measured as a diameter of the tumor.

Treating cancer may further result in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. The number of tumors may be measured by counting tumors visible to the naked eye or at a specified magnification. Preferably, the specified magnification is 2x, 3x, 4x, 5x, 10x, or 50x.

Treating cancer can result in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%.

Treating and/or preventing cancer can result in an increase in average survival time of a population of individuals treated according to the present invention in comparison to a population of untreated individuals. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with the product of the present invention. An increase in average survival time of a population may also be measured, for example, by calculating for a population the average length of survival following completion of a first round of treatment with the product of the present invention.

Treating and/or preventing cancer can also result in a decrease in the mortality rate of a population of treated individuals in comparison to an untreated population. Preferably, the mortality rate is decreased by more than 2%; more preferably, by more than 5%; more preferably, by more than 10%; and most preferably, by more than 25%. A decrease in the mortality rate of a population of treated individuals may be measured by any reproducible means, for example, by calculating for a population the average number of disease-related deaths per unit time following initiation of treatment with the product of the present invention. A decrease in the mortality rate of a population may also be measured, for example, by calculating for a population the average number of disease-related deaths per unit time following completion of a first round of treatment with the product of the present invention.

A further embodiment of the present invention relates to a method for preventing cancer recurrence by means of administering the product of the present invention. Cancer recurrence is a re-development of the cancer in an individual, who had previously undergone a cancer treatment, after a period of time in which no cancer could be detected. The probability of a cancer recurring depend on many factors, including the type of cancer and its extent within the body at the time of the treatment.

Some embodiments of the present invention are directed to the prevention and/or treatment of lung cancer.

Lung cancer can include all forms of cancer of the lung. Lung cancer can include malignant lung neoplasms, carcinoma *in situ,* typical carcinoid tumors, and atypical carcinoid tumors. Lung cancer can include small cell lung cancer ("SCLC"), non-small cell lung cancer ("NSCLC"), non-squamous non-small cell lung cancer, squamous non-small cell lung cancer, squamous cell carcinoma, non-squamous cell carcinoma, adenocarcinoma, small cell carcinoma, large cell carcinoma, adenosquamous cell carcinoma, and mesothelioma. Lung cancer can include "scar carcinoma", bronchioalveolar carcinoma, giant cell carcinoma, spindle cell carcinoma, and large cell neuroendocrine carcinoma. Lung cancer can include lung neoplasms having histologic and ultrastructual heterogeneity (e.g. mixed cell types).

Some other embodiments relate to the use of the product of the present invention for prevention and/or treatment of precancerous conditions of the lung. The term "precancerous conditions in the lung" as used therein refers to a group of cell proliferative disorders of the lung. Cell proliferative disorders of the lung include all forms of cell proliferative disorders affecting lung cells. Cell proliferative disorders of the lung can include hyperplasia, metaplasia, and dysplasia of the lung. Cell proliferative disorders of the lung can include asbestos-induced hyperplasia, squamous metaplasia, and benign reactive mesothelial metaplasia. Cell proliferative disorders of the lung can include replacement of columnar epithelium with stratified squamous epithelium, precancerous lung lesion and mucosal dysplasia. Individuals exposed to inhaled injurious environmental agents such as cigarette smoke and asbestos may be at increased risk for developing cell proliferative disorders of the lung. Prior lung diseases that may predispose individuals to development of cell proliferative disorders of the lung can include chronic interstitial lung disease, necrotizing pulmonary disease, scleroderma, rheumatoid disease, sarcoidosis, interstitial pneumonitis, tuberculosis, repeated pneumonias, idiopathic pulmonary fibrosis, granulomata, asbestosis, fibrosing alveolitis, and Hodgkin's disease.

The product of the present invention is further directed at individuals at risk of developing lung cancer. Such risk may be based on the medical or social history of an individual, such as inhalation of tobacco products as it occurs for example in smokers or exposure to asbestos or in non-smokers who breathe in secondhand smoke. Another category of individuals at risk for lung cancer are those harboring genetic mutations predisposing them to lung cancer. Yet another category is individuals who have been exposed to ionizing radiation or chemotherapeutic agents. Finally, another category is individuals with a known cancer at a location other than the lungs that have a propensity to metastasize to the lungs.

In another preferred embodiment, the invention relates to the products described herein for use in the prevention and/or treatment of brain cancers and/or precancerous conditions thereof. The term "brain cancer" as used herein refers to both primary brain tumors and metastatic brain tumors that originate from non-brain cancer cells such as lung cancer cells. Preferably, the term "brain cancer" refers to primary brain tumors.

Primary brain tumors are categorized by the type of tissue in which they first develop. The most common brain tumors are called glioma; they originate in the glial tissue. There are a number of different types of gliomas: for instance, astrocytomas, brain stem gliomas, ependymomas, and oligodendrogliomas.

Other types of primary brain tumors which do not originate from the glial tissue are, for instance, meningiomas, craniopharyngiomas and germinomas.

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### EXAMPLES

### Example 1: Aerosol administration of phospho-sulindac (PS V) prevents non-small cell lung cancer

The following example illustrates the efficacy of PS V administered by inhalation in preventing lung cancer.

**Inhalation exposure system:** The inhalation of PS **V** was carried out by using the arrangement as described in the US provisional application titled "COMPOUNDS AND COMPOSITIONS FOR USE IN THE TREATMENT AND PREVENTION OF LUNG AND BRAIN CANCER AND PRECANCEROUS CONDITIONS THEREOF".

PS V was dissolved in ethanol. PS solution in the baffle was aerosolized with the ultrasonic atomizer. The aerosol passed through an ascending stainless steel column, followed by a reflux column which was maintained at a temperature gradient by a heating tape (82 °C) and a chiller (5 °C) to condense and remove ethanol. PS aerosol exiting the reflux column was then passed through a charcoal column, which served to remove residual traces of ethanol from aerosol before it entered the animal-holding chamber. Experimental animals were held in nose-only air-tight tubes for designated time intervals.

**Orthotopic lung cancer model:** BALB/c nude mice (7 weeks old) were divided into control and treatment groups (15 mice/group) and treated following a prevention protocol by administration of either aerosol generated from ethanol (control) or PS **V** solution (treatment) for one week. The optimized exposure time and dose to mice were 50 mg/mL PS V for 8 min, respectively. On day 1 of week 2, a small incision (-5 mm) was made to the left side of the chest of anesthetized mice and 1 million GFP-A549 human lung cancer cells (A549 cells expressing green fluorescence protein (GFP) which allows their detection and quantification) were injected into their left lung as described by Doki, Y., et al. (Br. J. Cancer, 79, 7-8, pages 1121-1126, 1999). Inhalation treatment was resumed 2 days post-surgery and continued for 6 weeks when mice were euthanized, and blood and lung tissues were collected. Luminosity of the GFP-A549 tumors was measured and the lungs were weighed.

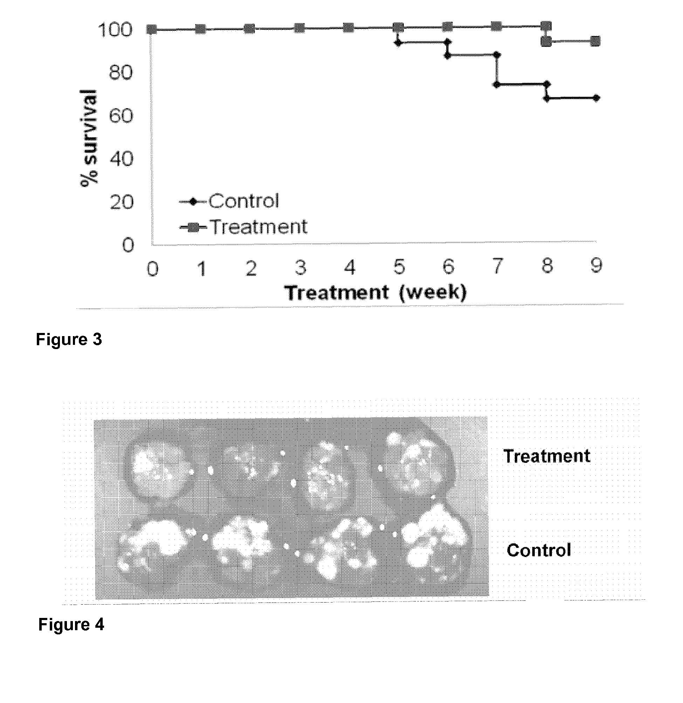

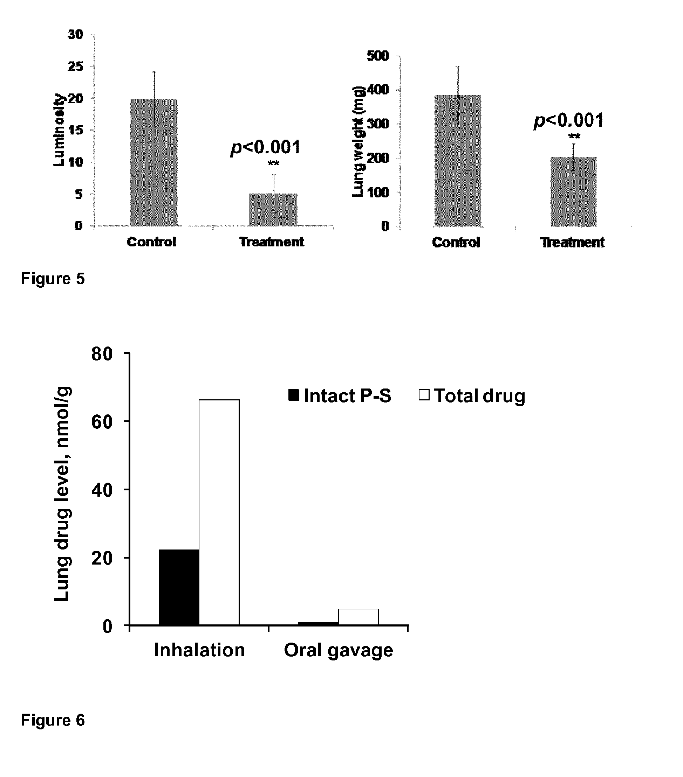

**Chemopreventive efficacy:** Two outcomes were used to gauge efficacy, animal survival and tumor size.
a) Survival: At the end of the study, 40 % of the mice in the control group died from the disease while the death rate in the treatment group was less than 10 % (p < 0.03). The results are illustrated by Figure 3.
b) Tumor size: At sacrifice, the tumor size was (all values, *Mean*±*SEM*) determined a) by luminosity: control = 19.85±4.33, treatment = 5.05±2.97 (p < 0.001). The results are shown in Figure 4 (upper photograph: after treatment; lower photograph: control group) and Figure 5 (left hand side); and b) by lung weight: control = 385.7±85.2 mg, treatment = 204.4±39.4 mg (p < 0.001). The results are shown in Figure 5 (right hand side).

### Example 2. The pharmacokinetic parameters of PS V after inhalational administration

PS **V** as well as sulindac, sulindac sulfide **XI** and sulindac sulfone **XII,** the structures of which are shown below, were administered to BALB/c nude mice.

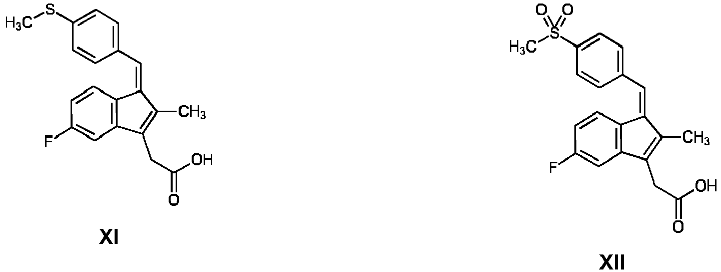

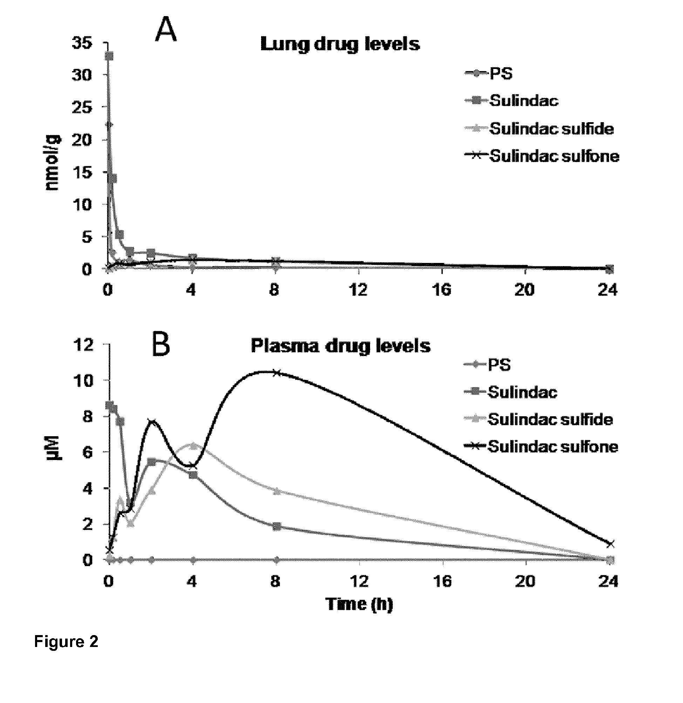

After 8 min of inhalation treatment, BALB/c nude mice were euthanized at various time points and drug levels were analyzed by HPLC in plasma and lung tissues. The results are summarized below and are further illustrated in Figure 2.

**Table 1**

| | Pharmacokinetic parameters in lung | | |
|---|---|---|---|
| | AUC | Cₘₐₓ, nmol/g | Tₘₐₓ, h |
| PS **V** | 7.7 | 22.2 | 0 |
| Sulindac | 30.1 | 32.9 | 0 |
| Sulindac sulfide **XI** | 18.9 | 1.4 | 4 |
| Sulindac sulfone **XII** | 57.5 | 4.6 | 8 |

**Table 2**

| | Pharmacokinetic parameters in plasma | | |
|---|---|---|---|
| | AUC | Cₘₐₓ, µM | Tₘₐₓ, h |
| PS **V** | 0 | 0 | - |
| Sulindac | 49.5 | 8.6 | 0 |
| Sulindac sulfide **XI** | 66.9 | 6.4 | 4 |
| Sulindac sulfone **XII** | 142.4 | 10.4 | 8 |

These findings indicate the following: a) inhalation provides intact PS V to the lungs, which is more cytotoxic to human cancer cells than either of its three metabolites, sulindac, sulindac sulfide **XI** and sulindac sulfone **XII;** b) oral administration does not provide intact PS **V** to the lungs, leading only to its three metabolites; and c) there are sufficient concentrations of sulindac and its metabolites in the circulation, and for prolonged periods of time. Sulindac, sulindac sulfide **XI** and sulindac sulfone **XII** are established cancer chemopreventive agents and thus, when derived from inhaled PS V, they can prevent smoking/nicotine-related cancers at sites other than the lung.

### Example 3: Inhalation delivery of aerosolized phospho-sulindac to the lungs of mice leads to higher drug levels than oral administration

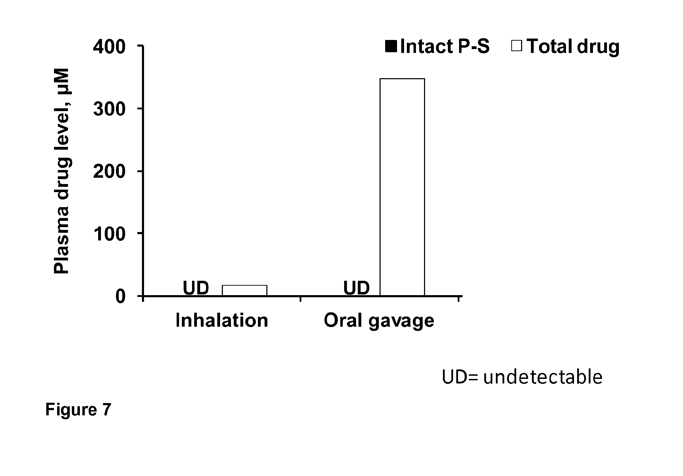

The delivery of aerosolized phospho-sulindac (PS **V**) to the lungs of mice was evaluated using the same inhalation device as in Example 1 and compared to its oral delivery. The PS **V** doses were: inhalational = 6.5 mg/kg body weight; oral = 150 mg/kg body weight. The level of PS **V** in the lungs and plasma after inhalation vs. after oral gavage are shown in Figure 6 and 7, respectively.

*Lungs:* PS levels: The aerosol-exposure system delivered a high level of intact PS **V** to the lungs of mice (> 20 nmol/g); while there were only trace levels of intact PS **V** (< 2 nmol/g) by oral administration. Total drug levels: It represents the total level of PS **V** plus its metabolites. The main metabolites of PS **V** are sulindac, sulindac sulfide **XI** and sulindac sulfone **XII;** at least the first two can cause gastrointestinal and renal side effects. The levels achieved by inhalation were significantly higher compared to those by oral administration.

***Plasma:*** PS levels: undetectable.Total drug levels after inhalation treatment (17 µM) were lower than after oral (348 µM) administration. Thus, inhalation delivery leads to blood levels of sulindac that can be chemopreventive for various non-lung cancers, but which are not particularly high so that can have significant potential toxicity. Of the three main metabolites of PS **V,** at least sulindac and sulindac sulfide **XI** can cause gastrointestinal and renal side effects.

Thus, PS **V** can be effectively delivered to lung cells by inhalation of a mixture of tobacco smoke with aerosolized PS **V.**

### Example 4. Curcumin enhances the lung cancer chemopreventive efficacy of phospho-sulindac V

Curcumin, the principal bioactive component in turmeric, exhibits anti-tumorigenic activities. In pre-clinical models of lung cancer, however, curcumin as a single agent has demonstrated poor efficacy (< 30 %). The present example demonstrates that curcumin potentiates the anti-cancer efficacy of PS **V** in the A549 human non-small cell lung cancer (NSCLC) cells, and that such a combination synergistically inhibited the growth of A549 xenografts in mice. These findings suggest that PS **V** in combination with curcumin is a promising combination therapy for the prevention of NSCLC.

Polymeric nanoparticles of poly(ε-caprolactone) (11000)-polyethylene glycol (5000) with entrapped curcumin were prepared according to the nanopercipitation-solvent displacement method. Four groups of female nude mice (n = 6 per group) at 7-8 weeks of age, were pre-treated for three days with 1) vehicle; 2) PS **V** 200 mg/kg/d; 3) curcumin 500 mg/kg/d; and 4) PS **V** 200 mg/kg/d plus curcumin 500 mg/kg/d. Then, the mice were inoculated subcutaneously on both flanks with A459 cells (2 x 10⁶ each). The treatment was resumed one day after tumor implantation and continued daily until the end of the study.

PS **V** alone produced a small inhibition of tumor growth that was statistically significant on days 12-22 after tumor implantation; whereas curcumin was ineffective for the duration of the study. On the other hand, PS **V** in combination with curcumin synergistically inhibited the growth of A549 xenografts, and the effect was statistically significant (p < 0.05) beginning on day 12 until the end of the study (day 36). At the end of the study, the average tumor volume of each group was as follows: control: 521 ± 76 mm³; PS **V:** 419 ± 36 mm³; curcumin: 599 ± 98 mm³; PS **V** plus curcumin: 290 ± 54 mm³. This corresponds to a reduction in tumor volume of 19.6 % and 44.3 % for PS **V** and PS **V** plus curcumin, respectively. In terms of tumor weight, a reduction was observed in the PS **V** (27 %, *p =* 0.06) and the PS **V** plus curcumin (51 %, p < 0.01) groups, but not in the curcumin-treated group. Of note, PS **V** plus curcumin treatment was significantly more effective than PS V or curcumin alone (p < 0.05).

### Example 5. Transdermal patch containing nicotine and an anti-cancer agent

The transdermal patch of the present invention can be manufactured analogously to the procedure disclosed in US 7,387,788.

Ethanol, propylene glycol, diethylene glycol monoethyl ether (and myristyl alcohol) are weighed and added successively. The mixture is homogenized using mechanical mixing. The resulting organic solution is clear and homogeneous. Nicotine hydrogen tartrate is added to 85-90% of the total amount of water and mixed until the solution is homogenized. Then the resulting aqueous solution is added to the organic solution, followed by an anti-cancer agent, such as phospho-sulindac V and mixed until homogenization of the solution is achieved. The resulting solution is clear and homogeneous. Then triethanolamine (typically about 50 wt.-% aqueous solution) is added and the solution mixed until the solution becomes homogeneous. The resulting solution is clear and homogeneous with a pH, for example, of about 6. When the pH is within the desired specification range water is added to the solution to obtain the desired weight percents (wt.-%) of the components and the pH of the final solution is measured. If the pH is below the desired pH (e.g. about pH 5.5), further triethanolamine solution is added and the pH of the final solution is remeasured. Typically, total triethanolamine amount does not exceed 5 wt.-%.

The composition of exemplary formulations 5.1-5.3 is summarized in **Table 3.**

**Table 3**

| Component | Composition (wt.-%) | | |
|---|---|---|---|
| | Formulation 5.1 | Formulation 5.2 | Formulation 5.3 |
| Nicotine tartrate | 2.85 | 2.85 | 2.85 |
| Absolute ethanol | 40.00 | 40.00 | 40.00 |
| Phospho-sulindac **V** | 5.00 | 2.00 | 1.00 |
| Diethylene glycol monoethyl ether | 5.00 | 5.00 | 5.00 |
| Propylene glycol | 15.00 | 25.00 | 25.00 |
| Myristyl alcohol | 1.00 | 1.00 | 0.00 |
| Hydroxypropylcellulose (Klucel HF) | 1.50 | 1.50 | 1.50 |
| Triethanolamine (50% w/w) | 5.07 | 3.52 | 4.00 |
| Purified water | 24.58 | 19.13 | 20.65 |

### Example 6. Cigarette containing an anti-cancer agent

The cigarette according to the present invention can be manufactured analogously to the procedure disclosed in US 2011/061667.

To 5 g of powdery agar (Wako Pure Chemical Industries, Ltd.) 100 ml of water is added, and the mixture is heated in a thermostat bath at 80 °C to dissolve agar. 25 g of I-menthol, 1.5 g fluorouracil and 2 ml of a 5 wt.-% aqueous solution of lecithin as an emulsifier are added thereto, and the mixture is sufficiently emulsified by means of a homogenizer. This emulsified slurry is cast on a substrate into a sheet form, which is dried in a forced air circulation dryer of 40 °C for one week. At this time, the emulsified state of the mixture is kept while the material is being dried.

The flavor-containing material for cigarette is blended in 5% by weight ratio to cut tobacco, and cigarettes with a tar value designed to about 10 mg are produced. The cigarettes may be optionally fitted with a plain filter.

### Example 7. Chewing gum containing nicotine and an anti-cancer agent

The chewing gum according to the present invention can be manufactured analogously to the procedure disclosed in US 2010/0130562. An example of a chewing gum composition is shown in Table 4.

**Table 4**

| Component | Composition (wt.-%) |
|---|---|
| Nicotine Polacrilex (18%) | 2.55 |
| Phospho-ibuprofen **I** | 2.00 |
| DREYCO^{®} gum base | 65.99 |
| Sorbitol | 22.1 |
| Fruit mint flavor | 3.8 |
| Sodium carbonate | 2.00 |
| Sodium bicarbonate | 1.00 |
| Acesulfame potassium | 0.25 |
| L-menthol | 0.25 |
| D&C Yellow 10 and Brown Lakes | 0.06 |

The composition is prepared by adding 1359.7 g of DREYCO^{®} gum base to a jacketed high shear mixer. The gum base is heated to about 60 °C and 50.9 g of Nicotine Polacrilex, 2.00 g of phospho-ibuprofen **I,** 442 g of sorbitol, 76 g of fruit mint flavor with ethanol as a carrier, 40 g of sodium carbonate, 20 g of sodium bicarbonate, 5.0 g of acesulfame potassium, 5.0 g of L-menthol and 0.8 g of D&C Yellow 10 and Brown Lakes are added. After the ingredients are mixed, the mixture is cooled to approximately 38 °C and removed from the mixer and then rolling and scoring process are performed to produce individual gum pieces. The gums are packaged into high density polyethylene bottles that are sealed and capped.

## Claims

1. A product comprising a nicotine-containing material and an anti-cancer agent.

2. The product according to claim 1, wherein the anti-cancer agent comprises a phospho-NSAID.

3. The product according to claim 1 or 2, wherein the anti-cancer agent comprises a compound selected from the group consisting of

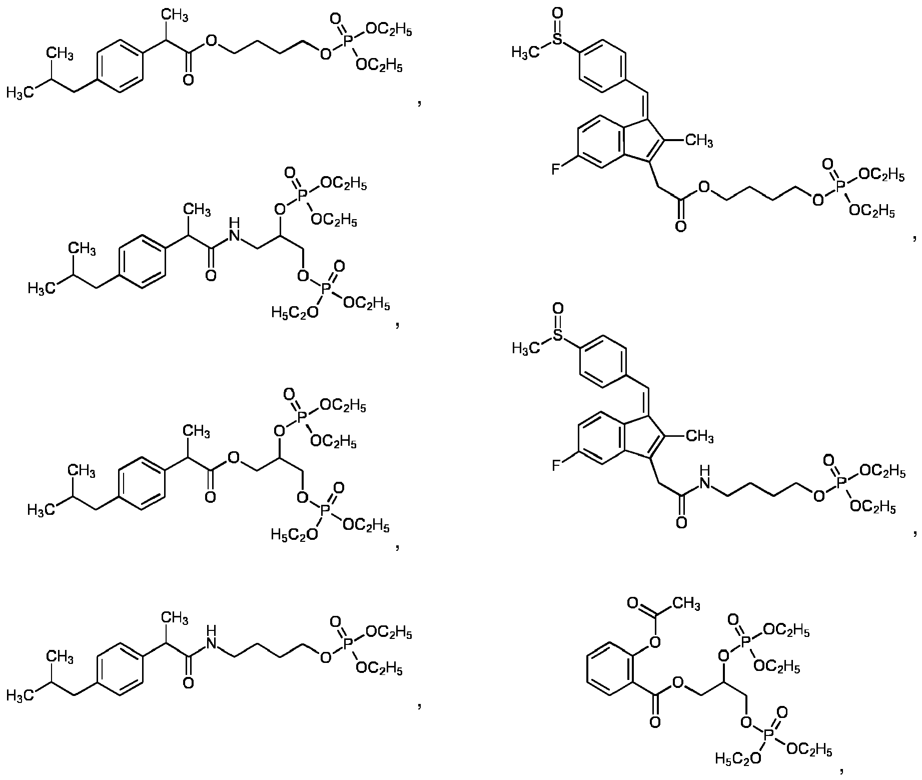

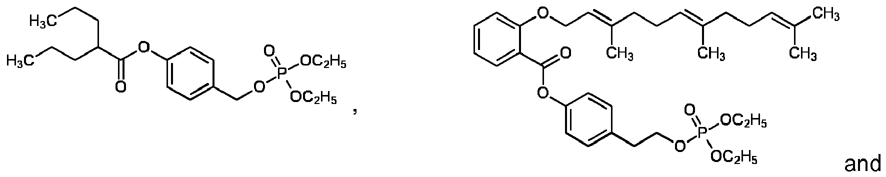

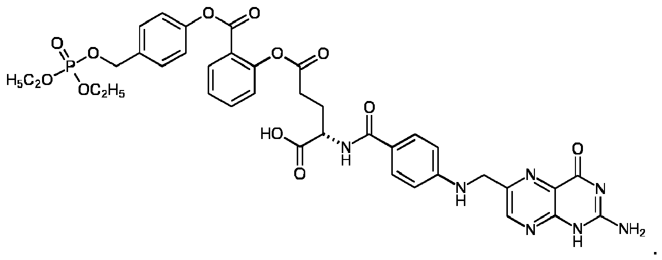

4. The product according to any of claims 1 to 3, wherein the anti-cancer agent is an oxidative stress enhancer.

5. The product according to any of claims 1 to 4, wherein the anti-cancer agent comprises a combination of at least two different compounds having anti-cancer activity, preferably a combination of the phospho-NSAID and curcumin.

6. The product according to any of claims 1 to 5, wherein the nicotine-containing material is tobacco leaf.

7. The product according to any of claims 1 to 6, wherein the product contains nicotine and the anti-cancer agent in the ratio of from 1000 : 1 to 1 : 10 (wt : wt).

8. The product according to any of claims 1 to 7, wherein the product is a smoking device selected from the group consisting of cigarette, cigar and smoking pipe, the smoking device optionally including an additional unit which renders the anti-cancer agent suitable for inhalation.

9. The product according to any of claims 1 to 7, wherein the product is a smoking cessation product, preferably a transdermal patch.

10. The product according to any of claims 1 to 7, wherein the product is an inhalation device, e.g. an electronic cigarette.

11. The product according to any of claims 1 to 7, wherein the product is an orally applied product.

12. The product according to claim 11, wherein the product is a smokeless tobacco product.

13. An anti-cancer agent for use in the prevention and/or treatment of cancer and/or precancerous conditions, wherein said anti-cancer agent is administered simultaneously with nicotine.

14. The anti-cancer agent for use according to claim 13, wherein the cancer is a lung cancer, brain cancer or a precancerous condition thereof.

15. The anti-cancer agent for use according to claim 13 or 14, wherein the anti-cancer agent is inhaled together with tobacco smoke.
